# EUROPÄISCHE PATENTSCHRIFT

(11) **EP 0 986 755 B1**
(45) Veröffentlichungstag und Bekanntmachung des Hinweises auf die Patenterteilung: **26.10.2005**
(21) Anmeldenummer: 98936100.1
(22) Anmeldetag: 04.06.1998
(51) Int. Cl.: G01N 33/53

(54) **STREPTAVIDIN/AVIDIN BESCHICHTETE OBERFLÄCHEN**
SURFACES COATED WITH STREPTAVIDIN/AVIDIN
SURFACES REVETUES DE STREPTAVIDINE/AVIDINE

(30) Priorität: 06.06.1997 DE 19724787
(43) Veröffentlichungstag der Anmeldung: 22.03.2000
(73) Patentinhaber: Biotez Berlin-Buch GmbH, 13125 Berlin (DE)
(72) Erfinder: STROHNER, Pavel, D-13187 Berlin (DE); IMMER, Ulrike, D-61279 Grävenwiesbach (DE)
(74) Vertreter: Baumbach, Friedrich
(86) Internationale Anmeldenummer: PCT/DE1998/001527
(87) Internationale Veröffentlichungsnummer: WO 1998/055864

(56) Entgegenhaltungen:
- EP-A- 0 151 492
- EP-A- 0 302 715
- EP-A- 0 331 127
- EP-A- 0 431 400
- EP-A- 0 762 122
- WO-A-91/07087
- WO-A-97/49989
- PATENT ABSTRACTS OF JAPAN vol. 016, no. 586 (P-1463), 25. Dezember 1992 & JP 04 236353 A (SHIN ETSU CHEM CO LTD), 25. August 1992
- STROHNER P ET AL: "Use of a modified binding model for the investigation of affinity dependence on antibody concentration in immunoassay systems" JOURNAL OF IMMUNOLOGICAL METHODS, Bd. 203, Nr. 2, 25. April 1997, Seite 113-122 XP004117422

## Beschreibung

Die Erfindung betrifft Streptavidin/Avidin beschichtete Oberflächen. Anwendungsgebiete der Erfindung sind die medizinische Diagnostik und die pharmazeutische Industrie.

Zur Bestimmung immunologisch wirksamer Komponenten nach dem Prinzip des Festphasen-Immunoassay, bei dem ein Reaktionspartner an eine feste Phase gebunden wird, verwendet man als Festphase heute üblicherweise Reaktionsgefäße in Form von Tubes oder Wells von Mikrotiterplatten, an deren Innenoberflächen der Reaktionspartner gebunden wird, bzw. Außenoberflächen von Kugeln. Das Ziel ist es, diese Oberflächen mit organisch chemischen Substanzen so zu beschichten, daß im nachfolgenden Assay höchste spezifische Bindungen erzielt werden und die unspezifischen Bindungen möglichst niedrig sind.

Zur Bestimmung einer spezifisch bindefähigen immunologischen Substanz, hat sich in den letzten Jahrzehnten der Immunoassay in seinen vielfältigen Formen etabliert. Er gibt die Möglichkeit zur Bestimmung kleinster Substanzmengen in Gegenwart millionenfachen Fremdstoffüberschusses. Grundlage ist die sensitive und - spezifische Konkurrenzreaktion einer unmarkierten Substanz P mit einer fixierten Menge an markierter Substanz P* um spezifische Bindungsstellen einer limitierten Menge eines Binders Q. Aus dem Verteilungsmuster des markierten Liganden in Abhängigkeit von der Konzentration an nicht markiertem Liganden kann die Konzentration des letzteren bestimmt werden. Zur Trennung von umgesetzten und nicht umgesetzten Reaktionspartnern sind verschiedenste Methoden entwickelt worden.

In den letzten Jahren hat die Festphasen-Methode, bei der ein Reaktionspartner an eine feste Phase gebunden wird, die Hauptbedeutung erlangt. Reaktionspartner, die an die feste Phase chemisch oder physikalisch gebunden werden, können Antikörper, Antigene, Rezeptoren, Zellen, DNA, RNA und vieles andere mehr sein. Diese Reaktionspartner können entweder direkt oder über eine Vorbeschichtung an die feste Phase adsorbiert werden. Heute werden sie üblicherweise mit einer spezifisch bindefähigen Substanz, dem Biotin, modifiziert, die es ermöglicht, an das beschichtete Trägermaterial zu binden. (Patent DE 36 40 412 A1 u.: Soukup, G.A. et al.: Bioconjugate Chemistry 6, 135/1995)

Als Trägermaterial werden heute üblicherweise Plast-Oberflächen aus Polystyrol, Polypropylen, Polyethylen, Polyamid, Polymethacrylat, Polycarbonat, Polyacrylat oder Copolymere von Polystyrol eingesetzt in Form von Innenoberflächen von Tubes oder Wells von Mikrotiterplatten oder Außenoberflächen von Kugeln.

Verschiedene Modifikationen dieser Oberflächen sind bekannt. Dabei muß die Bindung der spezifisch bindenden Substanz so erfolgen, daß die Anbindung des mit Biotin modifizierten Reaktionspartners stabil, unbeeinflußt und ausreichend ist für alle im Laufe des Bestimmungsverfahrens notwendigen Manipulationen. Der modifizierte, spezifisch bindende Reaktionspartner darf dabei seine Fähigkeit zur Bindung nicht verlieren. Die meisten Beschichtungen von festen Phasen beruhen heute auf adsorptiven Bindungen.
Zur Sicherung der Stabilität und optimalen Beschichtung wurden die adsorptive Bindung vermittelnde Klettsubstanzen vorgeschlagen. Dabei ist zu beachten, daß der spezifisch bindende Reaktionspartner in seiner Bindefähigkeit nicht beeinträchtigt wird (Patent DE 38 06 431).

Die bisher bekannten Nachweisverfahren haben folgende Nachteile
- die adsorptive Beladung mit Reaktionspartnern hat ein oft zu geringe Beladung zur Folge,
- zu geringe Aufnahmekapazität der beschichteten Oberflächen für die Bindung der nachfolgenden biotinylierten Reaktionspartner,
- oftmals auftretende hohe unspezifische Bindung,
- Instabilität gegenüber aggressiven Stoffen wie Detergenzien, starken Basen oder Säuren.

Davon ausgehend war es das Ziel der Erfindung, eine beschichtete Festphase zu entwickeln, die hohe Bindungsausbeuten an der Oberfläche liefert sowie genügend Stabilität, auch gegen Detergenzien, aufweist, die universell einsetzbar ist und dabei die notwendige Empfindlichkeit der durchzuführenden Bestimmungsverfahren ermöglicht.

Das Ziel der Erfindung wird gemäß Patentanspruch 1 und 17 erreicht, die Unteransprüche sind vorzugsvarianten.

Das Trägermaterial wird in einem ersten Schritt mit einer hydrophoben Klettsubstanz adsorptiv beladen, die ihrerseits modifiziert ist mit einem spezifischen Bindepartner für eine weitere, spezifisch bindende Substanz. Die Klettsubstanz kann ein Polypeptid, ein Protein, ein Kohlehydrat oder Glykoprotein sein. Sie kann unvernetzt, vernetzt oder derivatisiert eingesetzt werden. Bevorzugt werden zur Hydrophobisierung und/oder Vernetzung Proteine mit einem Molekulargewicht von 10 000 bis 900 000 benutzt. Besonders bevorzugt sind RSA, Lipase oder Immunglobuline wie Immunglobulin-gamma. Die Klettsubstanz muß hydrophober als das Trägermaterial sein.

Erfindungsgemäß ist die Klettsubstanz mit einem Bindepartner modifiziert, dem Biotin. Dies wird erfindungsgemäß ausgenutzt, eine stabile Bindung mit einer weiteren spezifisch bindefähigen Substanz, dem Streptavidin und Avidin herzustellen, was zu einer stabilen beschichteten festen Phase neuer Qualität führt. Streptavidin und Avidin besitzen mehrere Bindungsstellen für Biotin und können so nachfolgend mit Biotin-modifizierten Reaktionspartnern eines Bestimmungsverfahrens binden. Dabei wird die starke Biotin-Streptavidin- und Avidin-Bindung auf der einen Seite und die Tatsache, daß Streptavidin bzw. Avidin vier Bindungsstellen für Biotin besitzen, auf der anderen Seite ausgenutzt. Überraschenderweise haben die beschichteten Oberflächen die Eigenschaft, daß, obwohl durch die Bindung an biotinylierte Klettsubstanz Bindungsstellen vom Streptavidin und Avidin verbraucht wurden, die verbleibenden freien Bindungsstellen für Biotin um so affiner mit dem nachfolgend zu bindenden, mit Biotin modifizierten Reaktionspartner reagieren. Dies führt zu einer äußerst stabilen und hochsensiblen Festphase, wodurch die auf dieser Festphase entwickelten Bestimmungsverfahren wesentlich sensitiver gestaltet werden können.
Das zeigt sich besonders beim Nachweis von biotinylierten Oligonukleotiden und DNA-Fragmenten, bei dem geringste Mengen erfaßt werden können. Die erfindungsgemäß eingesetzte beschichtete Festphase ist in der Lage, sensibel auf kleinste Differenzen zu reagieren.

Das Klettprotein kann unvernetzt als Einzelmolekül, vernetzt oder derivatisiert verwendet werden. Die Methoden zur Herstellung sind dem Fachmann bekannt, ebenso die Methode der Biotinylierung. Wesentlich für die Realiserung der Erfindung ist eine Abstimmung des Vernetzungs- und des Biotinylierungsgrades, die Wahl der entsprechenden Klettsubstanz, Menge der Klettsubstanz und der des Streptavidins und Avidins zueinander und zur biotinylierten Klettsubstanz. Eine besonders bevorzugte Ausführungsform als Klettprotein ist Immunglobulin-gamma. Es kann sowohl unvernetzt als auch bevorzugt vernetzt mit den bekannten Spacern eingesetzt werden.

Als Faustregel gilt, je höher der Vernetzungs- und Biotinylierungsgrad, desto weniger Klettprotein ist einzusetzen. Wichtig ist, daß nicht zu viele Biotin-Bindungstellen am Streptavidin und Avidin besetzt werden, sonst kehrt sich der Effekt um. Die Menge Streptavidin/Avidin muß auf die Menge des biotinylierten Klettproteins abgestimmt werden.

**Tabelle:**

| eingesetzte Vernetzungsmittel | |
|---|---|
| Kurzform | chemische Bezeichnung |
| SPDP | N-Succinimidyl-3-(2-pyridylditho)-propionat |
| DSS | Disuccinimidylsuberat |
| DMS | Dimethylsuberimidat |
| MHS | Malimidohexanoyl-N-Hydroxysuccinimidester |
| MABI | Methyl-4-azidobenzoimidat * HCL |
| SAMBA | S'-Acetyl-mercaptobernsteinsäureanhydrid |
| MBS | m-Maleinimidobenzoyl-N-hydroxysuccinimidester |
| SATP | N-Succinimidyl-S-acetylthiopropionat |
| SATA | N-Succinimidyl-S-Acetylthioacetat |
| SADP | N-Succinimidyl-(4-azidophenyl)-1,3'-dithiopropionat |

Nach der Vernetzung wird vorzugsweise das Immunglobulin gamma biotinyliert nach den üblichen Methoden.

**Tabelle:**

| eingesetzte Biotinylierungsreagenzien | |
|---|---|
| NHS-Biotin | Biotin-Hydrazid |
| NHS-LC-Biotin | Biotin-LC-Hydrazid |
| Sulfo-NHS-LC-Biotin | Biotinamido-pentylamin |
| NHS-SS-Biotin | |

Der Biotinylierungsgrad bewegt sich erfindungsgemäß in einem Bereich zwischen 10 - 35. Die eingesetzten Mengen an vernetztem, biotinylierten Klettprotein liegen zwischen 5 - 15 µg/ml Beschichtungslösung je nach Biotinylierungsgrad.
Nachfolgend wird die Biotin-Streptavidin- und Avidin-Bindung auf der festen Phase durchgeführt. Die Mengen bewegen sich je nach Vorbeladung mit dem Klettprotein zwischen 2.5 und 20 µg/ml. Anschließend wird die feste Phase nach den dem Fachmann bekannten Methoden stabilisiert. Sie kann jetzt über Monate aufbewahrt werden, ist universell einsetzbar für alle Bestimmungsverfahren, in denen ein Reaktionspartner mit Biotin modifiziert wurde. Die beschichtete feste Phase ist so eingestellt, daß sie für alle bekannten Verfahren Verwendung finden kann. In einer besonderen Ausführungsform ist die feste Phase so ausgebildet (Wells von Mikrotiterplatten), daß sie gleichzeitig als photometrische Küvette dient. So kann die Messung der zu bestimmenden Substanz an der Oberfläche nach Ablauf der Reaktion im gleichen Gefäß erfolgen.

Das Verfahren führt zu hohen Signalausbeuten auf der beschichteten Festphase unter Minimierung der unspezifischen Bindungen. Die Festphase ist stabil gegen Einflüsse von Detergenzien. Deshalb kann die entwickelte Festphase bevorzugt in solchen Bestimmungsverfahren eingesetzt werden, wo dies eine besondere Rolle spielt. Es eignet sich vorzüglich in der RNA- und DNA-Diagnostik, wobei dafür Streptavidin/Avidin-Gemische besonders bevorzugt sind.

Die Erfindung soll nachfolgend durch Ausführungsbeispiele näher erläutert werden.

### Ausführungsbeispiele

### Beispiel 1 Thyreoglobulin-ELISA (TG-ELISA) an der beschichteten Fetsphase

Der TG-ELISA ist ein enzymimmunometrischer Assay, der aus einem biotinylierten Fänger-Antikörper (monoclonal), einem TG-Standard und einem POD-markierten Signal-Antikörper (monoclonal) besteht. Die Trennung erfolgt an der beschriebenen beschichteten festen Phase (Wells von Mikrotiterplatten (MTP)). Beide Antikörper binden an unterschiedlichen Bindungsdomänen des TG, so daß TG eine spezifische Brücke zwischen beiden Antikörpern (sandwich) bildet.

### Beschreibung:

Zweihundert Mikroliter einer biotinylierten, mit DSS vernetzten Immunglobulin-gamma (huIgG)) Lösung in 0.1M Karbonatpuffer pH 9.6 (vernetzt mit einem 20fachen Überschuß an DSS und biotinyliert mit einem Biotinylierungsgrad von 15) werden für 30 min. in die Wells einer MTP gegeben. Nach 2x Waschen mit 0.9% NaCl inkubieren die Wells in einem zweiten Schritt mit zweihundert Mikroliter einer Lösung aus 10 µg Streptavidin und 10µg Avidin pro Milliliter 0.1M Karbonatpuffer pH 9.6. Nach vier stunden wird 2x mit 0.9% NaCl gewaschen, für 30 min. mit 0.1% Casein nachgeblockt und weitere 30 min. mit 5% Denhardt scher Lösung in 0.1M Mc Ilvaine-Puffer pH 6 (0.05% RSA) geblockt. Zuletzt wird abgesaugt und die MTP über Nacht getrocknet. Die so beschichteten Platten werden bis zu ihrer Verwendung mit Trockenmittel eingeschweißt in Folie bei 4°C aufbewahrt.
Für den TG-Assay wird die MTP mit 200 µl biotinyliertem Fänger-Antikörper über Nacht beladen und anschließend mit 0.2% Trockenmilch für 30 min geblockt. Zur Assaydurchführung läßt man 100µl Mc Ilvaine-Puffer pH 6 (0.5% RSA), 50µl TG-Standard in einer Verdünnungsreihe bzw. 50µl Probe unbekannter Konzentration und 50µl Signal-Antikörper-POD mit dem an die feste Phase gebundenen Antikörper reagieren. Nach 4 h werden der umgesetzte Komplex und die nicht umgesetzten Reaktionspartner durch Absaugen getrennt. TG bindet in der Menge an die Oberfläche, in der es in der Lösung vorhanden ist. Der Signal-Antikörper-POD erkennt die gebundene Menge TG. Über eine nachgeschaltete Substrat-POD -(Horseradishperoxidase-Enzym) Reaktion kann die gebundene TG-Menge bestimmt werden. Als Substrat wird O-Phenylendiamin (OPD) verwendet. Die Höhe des Signals ist proportional der Menge vorhandenen TG's. Unbekannte Proben werden anhand ihrer Signalhöhe aus einer Eichkurve bestimmt.

### Beispiel 2: Hybridisierung zweier Oligonucleotide komplementärer Struktur auf der beschichteten Oberfläche (artifizielles System)

Zweihundert Mikroliter einer biotinylierten, mit DSS vernetzten Immunglobulin-gamma (huIgG)) Lösung in 0.1M Karbonatpuffer pH 9.6 (vernetzt mit einem 20fachen Überschuß an DSS und biotinyliert mit einem Biotinylierungsgrad von 15) werden für 30 min. in die Wells einer MTP gegeben. Nach 2x Waschen mit 0.9% NaCl inkubieren die Wells in einem zweiten Schritt mit zweihundert Mikroliter einer Lösung aus 10 µg Streptavidin und 10µg Avidin pro Milliliter 0.1M Karbonatpuffer pH 9.6. Nach vier Stunden wird 2x mit 0.9% NaCl gewaschen, für 30 min. mit 0.1% Casein nachgeblockt und weitere 30 min. mit 5% Denhardt'scher Lösung in 0.1M Mc Ilvaine-Puffer pH 6 (0.05% RSA) geblockt. Zuletzt wird abgesaugt und die MTP über Nacht getrocknet. Die so beschichteten Platten werden bis zu ihrer Verwendung mit Trockenmittel eingeschweißt in Folie bei 4°C aufbewahrt.
Auf eine beschichtete MTP werden 50µl eines biotinylierten Oligonucleotid-Einzelstranges (80mer) steigender Konzentration aufgebracht für 30 min bei 37°C. Anschließend wird einmal mit 0.9% NaCl-Tween (0.1%) gewaschen und mit 50µl eines FITCmarkierten Signalstranges (18mer) hybridisiert. Danach wird erneut gewaschen mit 0.9% NaCl-Tween (0.1%).Zum Nachweis der Hybridisierung werden 100µl einer Mischung aus anti-FITC-Antikörper (monoclonal) und einem zweiten anti-Maus-Antikörper-POD zu gleichen Teilen für 15min zugegeben. Danach erfolgt die Enzym-Substrat-Reaktion mit 50µl TMB -Lösung (Tetramethylbenzidin). Aus der Eichkurve mit steigenden Mengen des biotinylierten Einzelstranges können unbekannte Konzentrationen einer biotinylierten Probe aus der Höhe des gemessenen Signals abgelesen werden.

## Patentansprüche

1. Streptavidin/Avidin beschichtete Oberflächen, **dadurch gekennzeichnet, daß** die Schicht aus Streptavidin und Avidin besteht, welche über eine biotinylierte Klettsubstanz an der Oberfläche eines festen Trägermaterials gebunden ist.

2. Streptavidin/Avidin beschichtete Oberflächen nach Anspruch 1, **dadurch gekennzeichnet, daß** die Klettsubstanz vernetzt oder nicht vernetzt ist.

3. Streptavidin/Avidin beschichtete Oberflächen nach Anspruch 1, **dadurch gekennzeichnet, daß** die Klettsubstanz derivatisiert ist.

4. Streptavidin/Avidin beschichtete Oberflächen nach Anspruch 1-3, **dadurch gekennzeichnet, daß** Polypeptide, Kohlehydrate, Proteine, Glykoproteine mit einem Molekulargewicht von hauptsächlich größer als 10 000 als Klettsubstanz eingesetzt werden.

5. Streptavidin/Avidin beschichtete Oberflächen nach Anspruch 1-4, **dadurch gekennzeichnet, daß** die Klettsubstanz RSA, Lipase oder ein Immmunglobulin ist.

6. Streptavidin/Avidin beschichtete Oberflächen nach Anspruch 1-5, **dadurch gekennzeichnet, daß** die Klettsubstanz Immunglobulin-gamma ist.

7. Streptavidin/Avidin beschichtete Oberflächen nach Anspruch 1-6, **dadurch gekennzeichnet, daß** der Vernetzungsgrad der Klettsubstanz zwischen 10 und 30 liegt.

8. Streptavidin/Avidin beschichtete Oberflächen nach Anspruch 1-6, **dadurch gekennzeichnet, daß** der Biotinylierungsgrad der Klettsubstanz zwischen 15 und 35 liegt.

9. Streptavidin/Avidin beschichtete Oberflächen nach Anspruch 1-8, **dadurch gekennzeichnet, daß** die Klettsubstanz in Beschichtungslösungen einer Konzentration zwischen 5 und 15 µg/ml eingesetzt wird.

10. Streptavidin/Avidin beschichtete Oberflächen nach Anspruch 1-9, **dadurch gekennzeichnet, daß** die Beladung mit Streptavidin und/oder Avidin mittels Beschichtungslösungen einer Konzentration zwischen 5 und 20 µg/ml erfolgt.

11. Streptavidin/Avidin beschichtete Oberflächen nach Anspruch 1-10, **dadurch gekennzeichnet, daß** ein Gemisch von Streptavidin und Avidin im Verhältnis 60:40 bis 40:60 eingesetzt wird.

12. Streptavidin/Avidin beschichtete Oberflächen nach Anspruch 1-11, **dadurch gekennzeichnet, daß** ein Gemisch von Streptavidin und Avidin im Verhältnis 50:50 eingesetzt wird.

13. Streptavidin/Avidin beschichtete Oberflächen nach Anspruch 1-12, **dadurch gekennzeichnet, daß** 5-10 µg/ml unvernetzte Klettsubstanz mit einem Biotinylierungsgrad von 35 eingesetzt werden.

14. Streptavidin/Avidin beschichtete Oberflächen nach Anspruch 1-12, **dadurch gekennzeichnet, daß** 10 µg/ml vernetzte Klettsubstanz (10-30facher Überschuß an Vernetzungsmittel, bevorzugt Disuccinimidylsuberat) mit einem Biotinylierungsgrad von 15-20 eingesetzt werden.

15. Streptavidin/Avidin beschichtete Oberflächen nach Anspruch 1-14, **dadurch gekennzeichnet, daß** die Klettsubstanz adsorptiv an die Oberfläche gebunden ist.

16. Streptavidin/Avidin beschichtete Oberflächen nach Anspruch 1-15, **dadurch gekennzeichnet, daß** die Klettsubstanz an die Wells von Mikrotiterplatten gebunden ist.

17. Verfahren zur Herstellung von Streptavidin/Avidin beschichteten Oberflächen, **dadurch gekennzeichnet, daß** die biotinylierte Klettsubstanz auf die Oberfläche gebracht und nach deren Adsorption in einem zweiten Schritt die Schicht aus Streptavidin und Avidin aufgebracht wird.

## Claims

1. Surfaces coated with streptavidin/avidin wherein the layer consists of streptavidin and avidin which is bonded on the surface of a solid supporting material through a biotinylated adhering agent.

2. Surfaces coated with streptavidin/avidin according to claim 1 wherein the adhering agent is cross-linked or not cross-linked.

3. Surfaces coated with streptavidin/avidin according to claim 1 wherein the adhering agent is derivatized.

4. Surfaces coated with streptavidin/avidin according to claims 1 - 3 wherein polypeptides, carbohydrates, proteins, glycoproteins with a molecular weight, in principle, bigger than 10 000 are used as adhering agent.

5. Surfaces coated with streptavidin/avidin according to claims 1 - 4 wherein the adhering agent is RSA, lipase or an immune globulin.

6. Surfaces coated with streptavidin/avidin according to claims 1 - 5 wherein the adhering agent is immune globulin gamma.

7. Surfaces coated with streptavidin/avidin according to claims 1 - 6 wherein the degree of cross-linkage of the adhering agent is between 10 and 30.

8. Surfaces coated with streptavidin/avidin according to claims 1 - 6 wherein the degree of biotinylation of the adhering agent is between 15 and 35.

9. Surfaces coated with streptavidin/avidin according to claims 1 - 8 wherein the adhering agent is used in coating solutions of a concentration between 5 and 15 µg/ml.

10. Surfaces coated with streptavidin/avidin according to claims 1 - 9 wherein loading with streptavidin and/or avidin is effected by means of coating solutions of a concentration between 5 and 20 µg/ml.

11. Surfaces coated with streptavidin/avidin according to claims 1 - 10 wherein a mixture of streptavidin and avidin is used in a ratio of 60 : 40 to 40 : 60.

12. Surfaces coated with streptavidin/avidin according to claims 1 - 11 wherein a mixture of streptavidin and avidin is used in a ratio of 50 : 50.

13. Surfaces coated with streptavidin/avidin according to claims 1 - 12 wherein 5 - 10 µg/ml of a non-cross-linked adhering agent with a degree of biotinylation of 35 are used.

14. Surfaces coated with streptavidin/avidin according to claims 1 - 12 wherein 10 µg/ml of a cross-linked adhering agent (a 10 - 30-fold excess of cross-linkage agent, preferably disuccinimidyl suberate) with a degree of biotinylation of 15 - 20 are used.

15. Surfaces coated with streptavidin/avidin according to claims 1 - 14 wherein the adhering agent is adsorptively bonded on the surface.

16. Surfaces coated with streptavidin/avidin according to claims 1 - 15 wherein the adhering agent is bonded on the wells of microtitration plates.

17. Method of preparing surfaces coated with streptavidin/avidin wherein the biotinylated adhering agent is applied onto the surface and after its adsorption layer consisting of streptavidin and avidin is applied in a second stage.

## Revendications

1. Surfaces revêtues de streptavdine/avidine **caractérisées par le fait que** la couche de revêtement soit constituée de SS et d'AA, lesquelles sont liées à la surface d'un matériau porteur solide par une substance d'accrochage biotinylisée .

2. Surfaces revêtues de streptavdine/avidine selon la revendication 1, **caractérisées par le fait que** substance la d'accrochage est réticulée ou non réticulée.

3. Surfaces revêtues de streptavdine/avidine selon la revendication 1, **caractérisées par le fait que** la substance d'accrochage est dérivatisée.

4. Surfaces revêtues de streptavdine/avidine selon la revendication 1 à 3, **caractérisées par le fait que** des polypeptides, hydrates de carbones, protéines, glycoprotéines ayant un poids moléculaire supérieur à 10.000 soient utiliés comme substance d'accrochage

5. Surfaces revêtues de streptavdine/avidine selon la revendication 1 à 4, **caractérisées par le fait que** substance d'accrochage est un RSA, une lipase ou une immunoglobuline.

6. Surfaces revêtues de streptavdine/avidine selon la revendication 1 à 5, **caractérisées par le fait que** substance d'accrochage est de l'immunoglobuline gamma.

7. Surfaces revêtues de streptavdine/avidine selon la revendication 1 à 6, **caractérisées par le fait que** le degrés de réticulation de la substance d'accrochage se situe entre 10 et 30.

8. Surfaces revêtues de streptavdine/avidine selon la revendication 1 à 6, **caractérisées par le fait que** le degrés de biotinylisation de la substance d'accrochage se situe entre 15 et 35.

9. Surfaces revêtues de streptavdine/avidine selon la revendication 1 à 8, **caractérisées par le fait que** substance d'accrochage est utilisée dans des solutions d'enduction d'une concentration entre 5 et 15 µg/ml.

10. Surfaces revêtues de streptavdine/avidine selon la revendication 1 à 9, **caractérisées par le fait que** l'enduction avec de la streptavdine et/ou de l'avidine est effectuée au moyen de solutions d'enduction d'une concentration entre 5 et 20 µg/ml..

11. Surfaces revêtues de streptavdine/avidine selon la revendication 1 à 10, **caractérisées par le fait qu'**on utilise un mélange de streptavdine et d'avidine dans un rapport ses situant entre 60:40 et 40:60.

12. Surfaces revêtues de streptavdine/avidine selon la revendication 1 à 11, **caractérisées par le fait qu'**on utilise un mélange de streptavdine et d'avidine dans un rapport de 50 :50.

13. Surfaces revêtues de streptavdine/avidine selon la revendication 1 à 12, **caractérisées par le fait que** l'on utilise une substance d'accrochage non réticulée dans une concentration entre 5 et 15 µg/ml ayant un degrés de biotinylisation de 35.

14. Surfaces revêtues de streptavdine/avidine selon la revendication 1 à 12, **caractérisées par le fait que** l'on utilise une substance d'accrochage réticulée dans une concentration entre 10 µg/ml (excédent de 10-30 fois d'agent de réticulation de préférence subérate de disuccinimidyle) ayant un degrés de biotinylisation de 15 à 20.

15. Surfaces revêtues de streptavdine/avidine selon la revendication 1 à 14, **caractérisées par le fait que** la substance d'accrochage est liée à la surface de manière adsorptive

16. Surfaces revêtues de streptavdine/avidine selon la revendication 1 à 15, **caractérisées par le fait que** la substance d'accrochage est liée aux cupules de plaques à microtitrage.

17. Procédé de fabrication de surfaces revêtues de streptavdine/avidine **caractérisé par le fait que** la substance d'accrochage biotinylisée est appliquée sur la surface et que après l'adsorption de celle-ci dans une seconde étape la couche de streptavdine/avidine est appliquée.
